(19) Europäisches Patentamt
European Patent Office
Office européen des brevets

(11) Publication number : **0 445 096 A1**

(12) **EUROPEAN PATENT APPLICATION**

(21) Application number : **91870030.3**

(22) Date of filing : **25.02.91**

(51) Int. Cl.$^5$ : **C07C 235/74**, C07C 231/24

(30) Priority : **26.02.90 US 484301**

(43) Date of publication of application : **04.09.91 Bulletin 91/36**

(84) Designated Contracting States : **AT BE CH DE DK ES FR GB GR IT LI LU NL SE**

(71) Applicant : **MONSANTO COMPANY**
**Patent Department 800 North Lindbergh Boulevard**
**St. Louis, Missouri 63167 (US)**

(72) Inventor : **Vanderlinde, William**
**50 Rae Court**
**Greve Coeur, Missouri 63146 (US)**

(74) Representative : **Ernst, Hubert et al**
**Monsanto Services International S.A., Patent Department, Avenue de Tervuren 270-272, Letter Box No. 21**
**B-1150 Brussels (BE)**

(54) **Process for preparing metal-free fatty peroxyacid precusors having amide moieties in the fatty chain.**

(57) A process for preparing metal-free precursors for amide containing organic peracids is disclosed wherein a polycarboxylic acid is esterfied with a low molecular weight alkyl alcohol followed by formation of an amide by reacting the ester with an amine to provide a monoamido carboxylic acid ester. The ester is recovered from the reaction mixture by a distillation procedure under high temperature and reduced pressure. The esters are converted to acids highly desireable as precursers for peracids useful as bleaches in laundry detergents.

EP 0 445 096 A1

# PROCESSS FOR PREPARING METAL-FREE FATTY PEROXYACID PRECURSORS HAVING AMIDE MOIETIES IN THE FATTY CHAIN

This invention relates to the preparation of fatty acids which are to be oxidized to provide bleaching compounds comprising fatty peroxyacids and salts thereof.

## BACKGROUND OF THE INVENTION

The discovery of highly stable organic peracid molecules is critical to the commercialization of detergent formulations containing peracid bleaches. Such bleaches have recently been discovered which are highly crystalline and have relatively high melting points. Also, it is highly important for highly stable bleaches to be prepared in a manner which eliminates, or at least minimizes contamination from metals. Metals or metal ions are particularly deleterious to peracids because they catalyze the decomposition of the peroxygen group.

Consequently, the detergent industry requires peracids which are highly stable, have high melting points and are conveniently manufactured in high volume. Because of their high melting points both the peracids and their precursors are typically purified by precipitation or crystallization techniques. Metal ions typically present in the crystallization media become trapped in the peracid crystals and become impurities which reduce the stability of the peracid. The amount of metal ion contamination is directly related to stability of the peracid.

A recent patent, U. S. 4,634,551 to Burns et al describes novel, relatively stable and high melting crystalline amide peracids. Generally, the precursors to these amide peracids, that is, the amido acids, were reported to have been prepared by the reaction of the appropriate acid chloride with the appropriate amine followed by precipitation of the resulting amido acid. Stability of these amide peracids are affected not only by metal contamination but also by the chloride impurity. Attempts to purify the peracid has proven inadequate to economically remove metals and chlorides. Even purification of the amine precurser is not adequate to provide an economical product of sufficient purity for use in preparing the peracid.

The peroxyacids found in U. S. Patent 4,634,551 are represented by the the formula

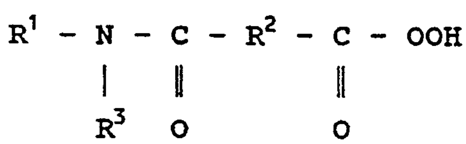

where in $R^1$ is selected from the groups consisting of alkyl, aryl or alkaryl radicals containing from about 1 to about 14 carbon atoms, $R^2$ is an alkylene group containing from 2 to 14 carbon atoms and $R^3$ is H or an alkyl, aryl or alkaryl group containing from 1 to about 10 carbon atoms, the total number of carbon atoms being from about 10 to about 20.

It is also known that organic acids have a tendency to scavenge metal ions in at least trace quantities. The presence of such metal ions, even in trace amounts, is undesirable in peracids because they catalyze the decomposition the peracid and can affect other properties such as melting point. A process for producing organic peracids is needed which avoids contamination with metal ions.

Accordingly, there is needed a process for preparing the fatty peroxyacids or salts thereof having an amide moiety in very high purity and with the avoidance of chloride ion.

## BRIEF DESCRIPTION OF THE INVENTION

In accordance with this invention there is provided a process for preparing precursors of fatty peroxyacids or salts thereof having amide moieties in the fatty chain. In one aspect of this invention, the above mentioned peroxyacid precursors are prepared by avoiding contamination with chloride ion therefore eliminating the need for extensive purification to remove such ion. In another aspect of this invention, the process of this invention provides the above mentioned peroxyacid precursors in a manner which greatly reduces contamination by trace metal.

More specifically, in accordance with this invention there is provided a process for preparing a monoamido ester of a dicarboxylic acid which comprises esterifying a polycarboxylic acid of the formula

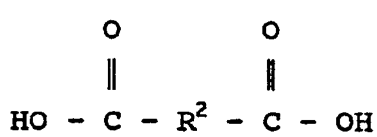

with a lower alkyl alcohol followed by reacting the ester with a monoalkylamine of the formula

$$RNH_2$$

wherein R is an alkyl radical having from 1 to 20 carbon atoms and $R^2$ is as defined above.

The reaction mixture obtained by the reaction of the ester and the amine is distilled under reduced pressure to remove the monoamide carboxylic acid ester from the reaction mixture. It has been found that such distillation efficiently separates the desired product from metal ions which remain in the residue. The process of this invention can provide metal-free amido esters and, when such esters are hydrolyzed

by conventional means, metal-free amido acids. The term "metal-free" as employed in this specification and claims means the total amount of all Transition Metals in the Periodic Table is below about .5ppm when the compound is analyzed by Inductively Coupled Plasma/Atomic Emission Spectroscopy (ICP/AES).

## DETAILED DESCRIPTION OF THE INVENTION

Typical dibasic acids include those having from 2 to 14 carbon atoms between the carboxyl groups. Preferrably the dibasic acids useful in this invention contain from about 6 to about 12 carbon atoms between the carboxyl groups and are aliphatic, straight chained. Included are adipic acid, glutaric acid, succinic acid, pimelic acid, suberic acid, azelaic acid, sebacic acid, and dodecanedioic acid.

Any number of alcohols may be employed to provide the diester. However, since the final product is a monoamide which is separated from a reaction mixture containing small amounts of unreacted diester, the alcohol is chosen so as to provide easily distinguishable properties as between the diester and monamide. Accordingly any suitable ester forming alcohol may be employed in the first step of the process of this invention. Examples of such alcohols are methanol, ethanol, propanol, isopropanol, n-butanol, hexanol, octanol, and other low molecular weight alkyl alcohols. Alkyl alcohols containing from about 1 to 4 carbon atoms are preferred and normal alcohols are preferred when higher molecular weight alcohols are employed. Methanol is preferred when the dibasic acid is adipic acid.

The catalyst employed in the esterification step is preferably toluene sulfonic acid. However, other inorganic acid catalysts may be employed. Such catalysts include phosphoric acid, sulfuric acid, etc. The use of such catalyst allows esterification at lower temperatures than uncatalyzed esterification. It has also been found that the esterification proceeds with greater efficiency at lower temperature, such as 80°C than uncatalyzed at 100°C. Also, the esterification proceeds with greater selectivity in the presence of a catalyst. A large number of acids are known to be useful as a catalyst and may be selected as the esterification catalyst. Other suitable inorganic acids include hydrochloric acid and phosphoric acid. Only a small amount of catalyst is required and the amount varies depending upon the dibasic acid employed. Usually the amount of catalyst employed is in the range of from .1% to about 1%, by weight of the dibasic acid. The catalyst enables the esterification reaction to proceed at a much lower temperature than otherwise possible.

It is usual that the dibasic acid is soluble in the alcohol and therefore the alcohol is employed as a diluent as well as a reactant. Also, it is desirable to remove water from the reactor both before and during the reaction. Constant removal is required because water is formed during esterification continuously. Removal is conveniently achieved by purging with alcohol at a temperature in excess of the boiling point of the reaction mixture. A mixture of water and alcohol is taken off overhead. When methanol is employed the reaction mixture is held at a temperature in the range of from about 95°C to about 125°C. For this reason, about a three-fold excess of methanol is employed. Recovery of the alcohol by means such as a reboiler and molecular sieve bed provides dry alcohol for reuse. The diester is recovered from the reaction mixture by any convenient means such as by vacuum distillation.

The reaction is economical in that the yield of diester is very high >95% in the case of dimethyl adipate) and the residue may be recycled.

In the next step of the process of this invention the diester is reacted with an amine to provide a distillable monoamide ester. The alcohol formed is removed and recycled. The reaction is monitored by GC analysis or by titration of the reaction mixture for residual amine. It is usual that the reaction providing the amide takes place almost instantaneously at higher temperatures and in about 1 to about 5 hours at a temperature in the range of from about 70°C to about 150°C. Usually the reaction between the amine and the ester is carried out with an excess of ester to maximize production of the desired monoamido and to minimize formations of diamide by product. Such excess is surprisingly large to provide the monoamide. Excess as high as 20 to 1 on a mole basis may be employed. In most instances the excess of diester over amine is in the range of from about 3 to 10 moles of diester per mole of amine. The mole ratio is preferably about 5 to 10 moles of diester to 1 mole of amine.

Amines employed in the process of this inventions are primary amines containing either straight or branched chain alkyl groups. Typically the amine contains from 1 to 20 carbon atoms and preferably from about 6 to about 12 carbon atoms. Such preferred amines are commercially available. Typical amines include hexylamine, heptylamine, octylamine, nonylamine, decylamine, dodecyclamine and undecyl amine. The linear or straight chain alkyl amines are preferred because the final amido acid has higher melting points than branched chained amido acids.

To provide high purity monoamide the reaction mixture is subjected to a two-step distillation procedure. The unreacted diester and traces of amine are first removed by distillation at relatively low temperature. Thereafter, the desired monoamide is distilled at high temperature under reduced pressure. It has been found that high vacuum enables the efficient separation of the desired product from undesired impurities, particularly metals. The term "reduced pressure" in this specification and claims means

pressure within the distillation column of no more than about 10mm Hg. Such reduced pressure is maintained during recovery of the desired monoamide. The temperature of the distillation depends upon the dibasic acid and the amido group. Usually, the temperature employed to distill the monoamide is in the range of from about 100°C to about 300°C but this depends upon the specific material being recovered. The diamide byproduct has been found to be usually a much higher boiling material than the monoamide thereby allowing efficient separation by distillation.

In one embodiment the diamide is hydrolyzed to recover the amine and both acid and amine reused in the process.

## DESCRIPTION OF THE PREFERRED EMBODIMENTS PREPARATION OF NONLYLAMIDO METHYL ADIPATE

Into a reactor fitted with an agitator, a distillation column, heating mantle and feed tube there was placed 1.7 moles of methanol and the reactor was purged and protected with nitrogen. With the agitator turned on .005 mole of sulfuric acid was added followed by .6 mole of adipic acid. The solution was heated to reflux with a condenser attached to the column to condense off gases. The temperature of the batch was allowed to rise to a range of 95°C to 105°C. When reflux slowed, 3.1 moles of methanol was slowly added over a period of 4 hours subsurface to maintain a steady flow from the condenser.

The temperature was maintained within the above stated range during the addition of the methanol and the acidity of the reaction mixture measured periodically. If the acidity was too high methanol was added and the reflux continued. At the completion of the reaction, as indicated by the acidity. The batch was heated to 130°C to remove residual methanol. The diester was recovered by distillation at a temperature in the range of 120°C - 125°C at a reduced pressure of 25mm Hg.

To a reactor equipped as described above there was added 1.3 moles of the diester prepared as described above which was then heated to 140°C and then .43 mole of nonylamine was added subsurface over a period of about 2 hours. The batch was held at that temperature for two additional hours after addition of the amine. Then, excess dimethyl adipate was removed by distillation at 140°C and reduced pressure (20-25mm Hg). The batch was then transferred to a high temperature high vacuum still and the portion distilled at 190-95°C at .5mm of Hg was collected. The receiver was warmed to about 50°C. About .25 mole of product was collected at a final pot temperature of not more than 220°C. By GC analysis, the product collected was determined to be 99.9% pure N-n-nonyl amido methyl adipate. The ester was hydrolyzed to the acid by conventional means and analyzed by ICP/AES which indicated the presence of Transition Metals to be below .5ppm. There was thus produced a metal free amido ester and amido acid. Obviously, the hydrolysis is conducted in a manner which avoids metal contamination.

The residue from the distillation operation was refluxed with 86g of 20% sulfuric acid for eight hours. About 25 ml of acetic acid was added to solubilize the residue. The resulting solution was diluted to about 250 ml with water, filtered, and neutralized with sodium hydroxide. The organic layer was extracted into ether, separated, and dried over sodium sulfate. After removal of the ether by evaporation, 10.86g remained which was indicated to be n-nonyl amine by analyses (GC). The amine can be recycled to prepare additional product.

There has been described above the preparation of amido esters of dibasic acids represented by the formula:

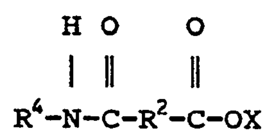

wherein $R^4$ is an alkyl group having from 1 to 20 carbon atoms, $R^2$ is an alkylene group containing from 2 to 14 carbon atoms and X is an alkyl radical having from 1 to 8 carbon atoms. These amido esters may be hydrolyzed in the usual way to provide metal-free amido acids represented by the formula:

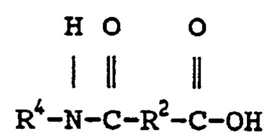

wherein $R^4$ and $R^2$ have the same meaning as indicated above. These amido acids have been found to be excellent precursor compounds of amido peracids because of the absence of metal ions.

## Claims

1. A process from the preparation of a monoamido ester of a dibasic acid represented by the formula

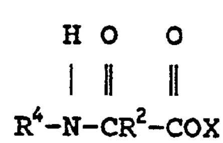

wherein $R^4$ is an alkyl group having from 1 to 20 carbon atoms, $R^2$ is an alkylene group containing

from 2 to 14 carbon atoms and X is an alkyl radical having from 1 to 8 carbon atoms which comprises the steps of:

(a) esterifying a polycarboxylic acid of the formula

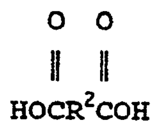

by reaction with an alkyl alcohol having from 1 to 8 carbon atoms;

(b) reacting the diester of the diacid of (a) with monoalkyl amine of the formula

$$RNH_2$$

wherein R is an alkyl radical having from 1 to 20 carbon atoms and $R^2$ is as defined above.

(c) distilling the monoamide produced in step (b) under reduced pressure to remove it from the reaction mixture.

**2.** The process of Claim 1 wherein the amine is n-nonylamine.

**3.** The process of Claim 2 wherein the alcohol is methanol.

**4.** The process of Claim 3 wherein the polycarboxylic acid is adipic acid.

**5.** The process of Claim 4 wherein the distillation of the monoamide is carried out under reduced pressure in the range of up to about 1 mm Hg.

**6.** The process of Claim 5 wherein the distillation temperature is in the range of from about 100°C to about 300°C.

**7.** The process of Claim 1 wherein the distillation is performed in at least two steps and in the first step the unreacted diester and amine are removed and in the second step the monoamide is removed.

**8.** The process of Claim 3 wherein methanol is also the solvent for the acid.

**9.** The process of Claim 1 wherein an acid catalyst is employed in the esterification step.

**10.** The process of Claim 9 wherein the acid catalyst is sulfuric acid.

**11.** The process of Claim 1 wherein the alcohol is an alkyl alcohol having from 1 to 4 carbon atoms.

**12.** The process of Claim 1 wherein the alcohol is an n-alkyl alcohol.

**13.** The process of Claim 1 wherein the alcohol is methanol.

**14.** The process of Claim 9 wherein the acid catalyst is selected from the group consisting of phosphoric acid and toluene sulfonic acid.

**15.** The process of Claim 1 wherein the dibasic acid is selected from the group consisting of succinic, glutaric, adipic, suberic and azealic acids.

**16.** The process of Claim 4 wherein the mole ratio of dimethyl adipate to n-nonylamine is in the range of from about 20 to 1 to about 1 to 1.

**17.** The process of Claim 16 wherein the mole ratio is in the range of from about 10 to 1 about 3 to 1.

**18.** The process of Claim 16 wherein the mole ratio is in the range of from about 10 to 1 to about 5 to 1.

**19.** The process of Claim 1 wherein the residue from step (c) is hydrolysed with an inorganic acid to provide monoalkyl amine.

**20.** The process of Claim 1 wherein the amine contains from 6 to 12 carbon atoms.

**21.** A metal-free amido ester represented by the formula

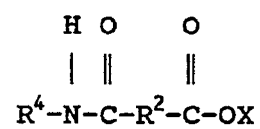

wherein $R^4$ is an alkyl group having from 1 to 20 carbon atoms, $R^2$ is an alkylene group having from 2 to 14 carbon atoms and X is an alkyl radical having from 1 to 8 carbon atoms.

**22.** An amido ester of Claim 21 wherein $R^4$ is an alkyl group having from 6 to 12 carbon atoms.

**23.** An amido ester of Claim 21 where $R^4$ is an alkyl group having 9 carbon atoms.

**24.** An amido ester of Claim 21 wherein $R^2$ is an alkylene group having from 2 to 8 carbon atoms.

**25.** An amido ester of Claim 21 wherein X is methyl.

**26.** A metal-free amido acid represented by the for-

mula

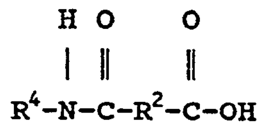

wherein $R^4$ is an alkyl group having from 1 to 20 carbon atoms, $R^2$ is an alkylene group having from 2 to 14 carbon atoms.

**27.** An amido acid of Claim 26 wherein $R^4$ is an alkyl group having from 6 to 12 carbon atoms and $R^2$ is an alkylene group having from 2 to 8 carbon atoms.

**28.** An amido acid of Claim 26 wherein $R^4$ has 9 carbon atoms and $R^2$ has four carbon atoms.

**29.** An amido acid of Claim 28 wherein $R^4$ is n-nonyl and $R^2$ is a straight chain alkylene group.

**30.** An amido ester of Claim 21 which is N-n-nonyl methyl adipate.

European Patent Office

# EUROPEAN SEARCH REPORT

Application Number

EP 91 87 0030

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-3 214 460 (HANSEL L. McGEE et al.) * the whole document * | 1-4,11-13,15 | C 07 C 235/74<br>C 07 C 231/24 |
| Y | | 1-4,6, 11-13, 15 | |
| Y | DE-C- 801 328 (BADISCHE ANILIN & SODA FABRIK) * the whole document * | 1-4,6, 11-13, 15 | |
| X | EP-A-0 260 134 (THE PROCTER & GAMBLE) * page 7, lines 7-20 * | 30 | |
| A | | 1,21-29 | |
| A | EP-A-0 170 386 (THE PROCTER & GAMBLE) * the whole document * | 1,26-29 | |
| A | US-A-4 676 933 (J.-J. LIN) * abstract * | 26 | |

| TECHNICAL FIELDS SEARCHED (Int. Cl.5) |
|---|
| C 07 C 235/00<br>C 07 C 231/00 |

The present search report has been drawn up for all claims

| Place of search | Date of completion of the search | Examiner |
|---|---|---|
| BERLIN | 08-05-1991 | RUFET J.M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)